# EUROPEAN PATENT SPECIFICATION

(11) **EP 3 772 946 B1**
(45) Date of publication and mention of the grant of the patent: **22.09.2021**
(21) Application number: 19729843.3
(22) Date of filing: 10.05.2019
(51) Int. Cl.: A01P 1/00, A01N 33/04, A01N 43/16, A01N 57/12, A61K 47/36, A61K 9/127, A61K 9/00, A61K 31/728

(54) **COMPOSITION HAVING ANTIMICROBIAL ACTIVITY**
ZUSAMMENSETZUNG MIT ANTIMIKROBIELLER WIRKUNG
COMPOSÉ AVEC DE L'ACTIVITÉ ANTIMICROBIENNE

(30) Priority: 11.05.2018 IT 201800005309
(43) Date of publication of application: 17.02.2021
(73) Proprietor: Offhealth S.p.A., 50144 Firenze (FI) (IT)
(72) Inventor: ZANINI, Alessandro, 56017 San Giuliano Terme (IT); VELLANTE, Marco, 62029 Tolentino (IT); SODO, Eugenio, 00125 Roma (IT); CAVALLO, Giovanni, 00122 Roma Ostia (IT); FOSCHINI, Fulvio, 00124 Roma (IT)
(74) Representative: Sarpi, Maurizio
(86) International application number: PCT/IB2019/053888
(87) International publication number: WO 2019/215700

(56) References cited:
- WO-A1-2015/170247
- WO-A1-2018/073720
- CN-A- 104 940 140
- CN-A- 105 726 485
- C.L. ROMANÒ ET AL: "Hyaluronic Acid and Its Composites as a Local Antimicrobial/Antiadhesive Barrier", JOURNAL OF BONE AND JOINT INFECTION, vol. 2, no. 1, 1 January 2017 (2017-01-01) , pages 63-72, XP055514825, ISSN: 2206-3552, DOI: 10.7150/jbji.17705
- ANDREA ARDIZZONI ET AL: "Influence of hyaluronic acid on bacterial and fungal species, including clinically relevant opportunistic pathogens", JOURNAL OF MATERIALS SCIENCE: MATERIALS IN MEDICINE, KLUWER ACADEMIC PUBLISHERS, BO, vol. 22, no. 10, 4 September 2011 (2011-09-04), pages 2329-2338, XP019961613, ISSN: 1573-4838, DOI: 10.1007/S10856-011-4408-2
- DRULIS-KAWA ZUZANNA ET AL: "A comparison of thein vitroantimicrobial activity of liposomes containing meropenem and gentamicin", CELLULAR & MOLECULAR BIOLOGY LETTERS, UNIVERSITY OF WROCAW. INSTITUTE OF BIOCHEMISTRY, WROCAW; PL, PL, vol. 11, no. 3, 1 January 1901 (1901-01-01), pages 360-375, XP035994422, ISSN: 1425-8153, DOI: 10.2478/S11658-006-0030-6 [retrieved on 1901-01-01]

## Description

### Field of the invention

The present invention relates to the medical-pharmaceutical field, and more specifically to the field of preparations for topical use which have antimicrobial activity which find application in a wide range of uses. For example, the composition can be applied in all those areas where it is necessary to eliminate or reduce the risk of infection caused by microbes on a wide range of surfaces, in particular on the epithelial surface.

### Background of the invention

Microbial infections represent an important global health risk affecting millions of people around the world. Over the years, many drugs and antimicrobial devices have been developed to improve the treatment or prevention of microbial infections. However, we are witnessing at this time a progressive loss of efficacy of the antibiotic therapies in use and the emergence of infections resistant to these drugs represents a potentially devastating damage for the collective health, the health systems, and the economies of the whole world. It is therefore necessary to counterattack as soon as possible, acting on two fronts: that of prevention, aimed at avoiding or containing the development of further resistance and the spread of infections, which is played on the ground of surveillance, correct use of antibiotics, and protocols in hospitals. The other front is the research and development of new active ingredients and compositions with such an activity.

Therefore, the need is strongly felt for new and alternative compositions with antimicrobial activity which allow widening the range of action against the microbial determinants of pathologies harmful for humans and animals.

US patent application US2013/0216606 relates to a liposomal formulation for administering drugs in the eye comprising (i) liposomes comprising at least one lipid bilayer and (ii) a prostaglandin drug and/or a prostaglandin derivative associated in liposomes, wherein the liposomes have an average diameter of less than 2µπ, and the pharmaceutical product comprising the liposomal formulation and a method for producing the liposomal formulation for the release of ocular drugs. In one embodiment thereof, the claimed pharmaceutical composition is in a form of a viscous aqueous carrier, wherein the viscous aqueous carrier comprises an aqueous solution of polysaccharides, wherein the polysaccharide is hyaluronic acid.

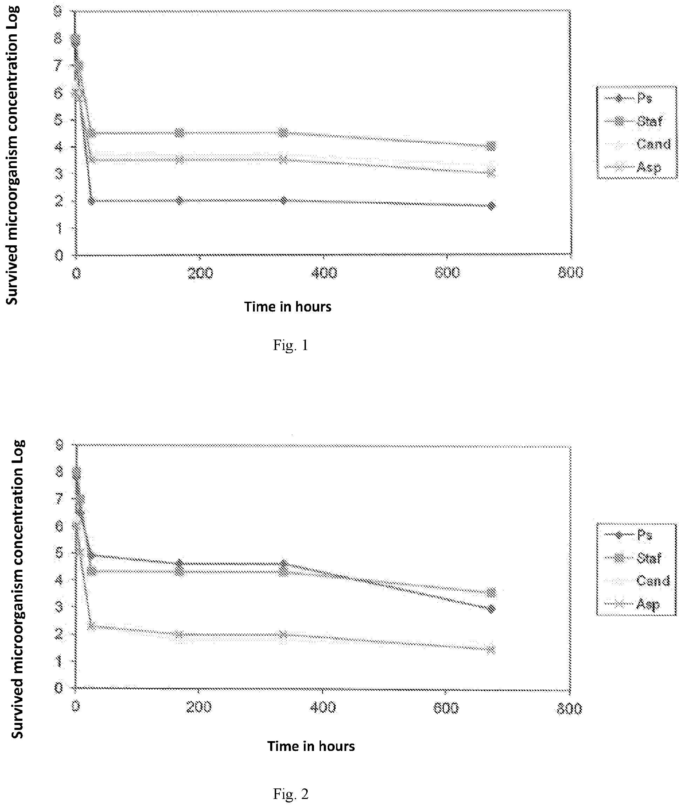

In literature, the article "Hyaluronic Acid and its Composites as a Local Antimicrobial /Antiadhesive Barrier" of the JOURNAL OF BONE AND JOINT INFECTION, vol.2, no.1, January 1, 2017 (2017-01-01) pages 63-72 reports a study of the antibacterial activity of hyaluronic acid as such, in particular focused on HA's ability to destroy biofilms. In particular, great potential is provided in the case of implants and tissues of the host. Adhesion studies are conducted in the case of S. aureus (see summary, figures 2-5).

The article "Influence of hyaluronic acid on bacterial and fungal species, including clinically relevant opportunistic pathogens" on JOURNAL OF MATERIALS SCIENCE: MATERIALS IN MEDICINE, KLUWER ACADEMIC PUBLKISHERS, BO, VOL.22, NO.10, September 4, 2011 , pages 2329 -2338), is a study on the antibacterial effect of hyaluronic acid as such against certain strains of different bacteria. Developmental inhibition is observed in vitro in many cases and is dose-dependent (see summary; figures 1-4).

Drulis-Kawa Zuzanna et Al. in thye article: "A comparison of the vitroantimicrobial activity of liposomes containing meropenem and gentamicin" published in CELLULAR & MOLECULAR BIOLOGY LETTERS, UNIVERSITY OF WROCAW. INSTITUTE OF BIOCHEMISTRY, WROCAW; PL, vol. 11, no.3 January 1901, pages 360-375, reports a study on the effect of drug encapsulation (meropenem and gentamicin) with liposomes which are positively or negatively charged or are neutral. Meropenem and Gentamicin are antibiotics whose activity is changed by the type of encapsulation. Cholesterol and phosphatidylcholine liposomes are preparations to support octadecylamine and the antibacterial effect of the antibiotic drugs carried by liposomes is observed.

Unlike the prior art, the present invention proposes the association of stearylamine in liposomes, and of hyaluronic acid, or a salt thereof, both in cross-linked and non-cross-linked form, of which the marked antimicrobial properties are shown, where the concentration of each component as well as the molar ratio between phospholipids and stearylamine in liposomes are well defined.

### Summary of the invention

The present invention first relates to the association of stearylamine in liposomes and of hyaluronic acid, or a salt thereof, both in cross-linked and non-cross-linked form.

The present invention secondly relates to such an association of stearylamine in liposomes and hyaluronic acid, or a salt thereof, both in cross-linked and non-cross-linked form, for use in the treatment of infections caused by microorganisms of various nature including bacteria, fungi and yeasts.

The present invention further relates to compositions comprising said association of stearylamine in liposomes and hyaluronic acid, or a salt thereof, both in cross-linked and non-cross-linked form, to be used in personal hygiene for disinfecting the body surface of humans and animals, for the disinfection of surfaces in a domestic environment and/or in food service areas, hospitals and in the treatment and prophylaxis of microbial infections.

In a particularly preferred embodiment, the composition comprises the association of stearylamine in liposomes and a cross-linked hyaluronic acid salt for use in the treatment and prophylaxis of ocular infections caused by microorganisms.

### Brief description of the drawings

Figure 1 shows the logarithmic diagram of the concentration of the inoculum pool of the strains analyzed in contact with the composition 1 comprising 0.005% w/w Stearylamine.
Figure 2 shows the logarithmic diagram of the concentration of the inoculum pool of the strains analyzed in contact with the composition 2 comprising 0.005% w/w Stearylamine with hyaluronic acid sodium salt.
Figure 3 shows the logarithmic diagram of the concentration of the inoculum pool of the strains analyzed in contact with the composition 3 comprising 0.005% w/w Stearylamine with cross-linked hyaluronic acid sodium salt.
Figure 4 shows the logarithmic diagram of the concentration of the inoculum pool of the strains analyzed in contact with the composition 1 comprising 0.005% w/w Stearylamine liposomes.
Figure 5 shows the logarithmic diagram of the concentration of the inoculum pool of the strains analyzed in contact with the composition 5 comprising 0.005% w/w Stearylamine liposomes with hyaluronic acid sodium salt.
Figure 6 shows the logarithmic diagram of the concentration of the inoculum pool of the strains analyzed in contact with the composition 3 comprising 0.005% w/w Stearylamine liposomes with cross-linked hyaluronic acid sodium salt.
Figure 7 shows the logarithmic diagram of the concentration of the inoculum pool of the strains analyzed in contact with the composition 7 comprising 0.00125% w/w Stearylamine liposomes and cross-linked hyaluronic acid sodium salt.
Figure 8 shows the logarithmic diagram of the concentration of the inoculum pool of the strains analyzed in contact with the composition 8 comprising 0.00125% w/w Stearylamine liposomes and hyaluronic acid sodium salt.
Figure 9 shows the logarithmic diagram of the concentration of the inoculum pool of the strains analyzed in contact with the composition 9 comprising 0.00125% w/w Stearylamine liposomes.
Figure 10 shows the logarithmic diagram of the concentration of the inoculum pool of the strains analyzed in contact with the composition 10 comprising 0.0025% w/w Stearylamine liposomes and cross-linked hyaluronic acid sodium salt.
Figure 11 shows the logarithmic diagram of the concentration of the inoculum pool of the strains analyzed in contact with the composition 11 comprising 0.0025% w/w Stearylamine liposomes and hyaluronic acid sodium salt.
Figure 12 shows the logarithmic diagram of the concentration of the inoculum pool of the strains analyzed in contact with the composition 12 comprising 0.0025% w/w Stearylamine liposomes.

### Detailed description of the invention

The following description relates in detail to some particularly preferred embodiments for the implementation of the invention, with particular reference to the large experimental section from which those skilled in the art can easily understand the considerable advantages related to and resulting from the use of the present invention.

The various embodiments of the present invention provide liposomal formulations containing stearylamine of various molar ratio between phospholipids and stearylamine, where, according to the invention, the active ingredient is given by the association of liposomes containing stearylamine with hyaluronic acid, or a salt thereof, preferably in cross-linked form, but, as will be shown by the experimental data presented below, the liposomal formulation containing stearylamine therein has an antimicrobial effect higher than non-liposome stearylamine, and furthermore the addition of hyaluronic acid salt allows obtaining an enhanced antibacterial and antifungal effect.

In the context of the present description, the expression "liposomal formulation" indicates a liposome formulation, where liposomes are artificially prepared vesicles consisting of double lipid layers which form a thin membrane consisting of two layers of lipid molecules; double lipid layers may be unique or multiple. These vesicular structures can be filled or loaded with drugs or active ingredients and used precisely for the transmission thereof in some districts otherwise difficult to reach. Therefore, the drug or active ingredient can be associated with liposomes, where the term "associated" generally refers to being coupled, linked, correlated, bound or encapsulated.

In the various embodiments, the liposomes may comprise lipids selected from the group of: fatty acids, glycerolipids, phospholipids, glycerophospholipids, sphingolipids, saccharolipids, polyketides sterols and prenols. Liposomes may consist of natural phospholipids with mixed lipid chains or other surfactants.

In a particularly preferred embodiment of the present invention, the lipids of the lipid formulation are phospholipids. Phospholipids are amphipathic molecules, i.e. organic compounds having a water-soluble phosphate-based polar head and a non-water-soluble apolar tail, i.e. not soluble in water and soluble in apolar solvents.

The interest of liposomes is clearly linked to the structural and, therefore, functional properties of their membrane, whose structure, composition and proportions are similar to those of the cell membrane. In an aqueous medium, the hydrophobic tails of the phospholipids attract each other, while the hydrophilic heads arrange themselves in contact with the outside and with the internal aqueous environment, with an arrangement similar to that of the micelles. Therefore, double lipid layers are formed which close, thus forming small vesicles similar to the cells of the organism and of the organelles thereof.

Such spheres or liposomes form small deposits which may contain an antigen, an antibiotic, an allergen, a drug or a gene (gene therapy) and be introduced into the body without causing immune rejection reactions. In particular, the liposoluble molecules will be housed in the double layer, the water-soluble molecules inside the liposome and the molecules with mixed properties, between the double layer and the inside.

Furthermore, the features of liposomes can be modified according to the different drugs conveyed. For example, in order to decrease the degradation rate of the liposome and slow down the release of the contents thereof, the composition and dimensions are modified. It is also possible to increase the affinity of the liposomes for a given tissue by changing their composition and electric charge. For example, according to the present invention, the addition of stearylamine gives the liposomal formulation a positive charge.

Hyaluronic acid is a high molecular weight polysaccharide of the glycosaminoglycan group. Hyaluronic acid consists of a linear chain consisting of disaccharide units of N-acetylglucosamine and glucuronic acid. Hyaluronic acid is one of the main components of connective tissue and has an important structural role in the skin, along with collagen and elastin. The main property of hyaluronic acid is to bind a high number of water molecules ensuring the hydration of the skin. Due to its high affinity with water, hyaluronic acid is used as a skin moisturizer. In fact, when applied to the skin, it forms a thin film which keeps it smooth and hydrated. Finally, the smaller molecules are able to penetrate the subcutaneous layers giving firmness to the tissues and causing a temporary filling and smoothing of small wrinkles.

Surprisingly, it has been found that the stearylamine liposomes associated with hyaluronic acid or a salified form of hyaluronic acid, in particular in cross-linked form, are provided with antimicrobial activity, preferably the salt is a sodium salt.

In the association according to the present invention, stearylamine ranges between 0.001 and 0.01% by weight, preferably between 0.0125 and 0.005% by weight, more preferably in the association according to the invention, stearylamine is present in a concentration equal to 0.025% by weight.

Hyaluronic acid, or salt thereof, preferably sodium salt, in the association according to the invention, is at a concentration ranging from 0.05 to 0.5% by weight, preferably from 0.1 to 0.3% by weight, more preferably in the association according to the invention, the hyaluronic acid salt is present at a concentration of 0.15% by weight. In a particularly preferred embodiment, hyaluronic acid is cross-linked.

In another aspect, the present invention relates to the composition comprising the liposomal formulation described above and pharmaceutically accepted excipients. Said composition may further comprise active ingredients, buffering agents, preservatives, diluents, carriers and wetting agents.

The technical features of the second composition are such as to allow sterilization by 0.2-micron filtration, making it suitable for all uses in which sterility is required.

The formulation of the composition according to the invention is given below by way of example:

### Example 1

| Ingredient | % w/w |
|---|---|
| Phospholipids E80 | 0.500 |
| Stearylamine | 0.001-0.010 |
| Cross-linked hyaluronic acid | 0.05-0.5 |

| | |
|---|---|
| Sodium chloride | 0.480 |
| Monobasic sodium phosphate dihydrate | 0.225 |
| Dibasic sodium phosphate dihydrate | 0.685 |
| Trehalose | 0.30 |
| Distilled water | q.s. to 100 g |

According to the formulation of example 1, the buffering capacity is provided by the monobasic/dibasic sodium phosphate system, however it will be apparent to those skilled in the art that in some embodiments according to the present invention other buffer systems known in the field may be used, such as for example the citrate or borate buffer system, which have been shown to provide adequate buffering capacity to maintain the pH value in the desired range, as in example 2 presented below.

### Example 2

| Ingredient | % w/w |
|---|---|
| Phospholipids E80 | 0.500 |
| Stearylamine | 0.001-0.010 |
| Cross-linked hyaluronic acid | 0.05-0.5 |
| Sodium chloride | 0.450 |
| Boric acid | 0.775 |
| Sodium tetraborate decahydrate | 0.125 |
| Trehalose | 0.30 |
| Distilled water | q.s. to 100 g |

According to a further aspect of the invention, the described composition is used by exploiting its antimicrobial activity, demonstrated in particular with respect to bacteria, fungi and yeasts; therefore, it can be useful in deep cleaning and personal hygiene for disinfecting the body surface of humans and animals, for the disinfection of surfaces in a domestic environment, in food service areas, in hospitals and the like, and for use in the treatment and prophylaxis of microbial infections.

In particular, another aspect of the present invention relates to pharmaceutical compositions comprising the liposomal formulation of stearylamine in association with hyaluronic acid, or a salt thereof, in particular in a cross-linked form, where the salt is sodium salt.

In a particularly preferred embodiment, such pharmaceutical compositions are in the form of an ophthalmic solution with antimicrobial activity for use in the treatment of eye infections caused by bacteria, fungi and yeasts, such as, by way of example, *Pseudomonas aeruginosa, Staphylococcus aureus, Aspergillus niger* and *Candida albicans.*

The presence of stearylamine makes the liposomal formulation, and therefore the pharmaceutical composition for ophthalmic use, positively charged and therefore particularly adapted to be retained on the surface of the corneal epithelium, which like all cell membranes is negatively charged. Therefore, the electrostatically attracted ophthalmic composition is retained on the ocular surface and able to perform better the delivery action and its therapeutic action. On the contrary, anionic compositions tend not to be integrated into the epithelium, but rather repulsed by the epithelium and remain in the tear film, thus being more effective as artificial tears or lipid film integrators.

By way of example, the formulations of the ophthalmic composition according to the present invention comprising the liposome formulation of stearylamine and cross-linked hyaluronic acid sodium salt are given in a particularly preferred embodiment.

### Example 3

| Ingredient | % w/w |
|---|---|
| Phospholipids E80 | 0.500 |
| Stearylamine | 0.0025 |
| Cross-linked hyaluronic acid | 0.15 |
| Sodium chloride | 0.480 |
| Monobasic sodium phosphate dihydrate | 0.225 |
| Dibasic sodium phosphate dihydrate | 0.685 |
| Trehalose | 0.30 |
| Distilled water | q.s. to 100 g |

### Example 4

| Ingredient | % w/w |
|---|---|
| Phospholipids E80 | 0.500 |
| Stearylamine | 0.0025 |
| Cross-linked hyaluronic acid | 0.05-0.5 |
| Sodium chloride | 0.450 |
| Boric acid | 0.775 |
| Sodium tetraborate decahydrate | 0.125 |
| Trehalose | 0.30 |
| Distilled water | q.s. to 100 g |

### Example 5

| Ingredient | % w/w |
|---|---|
| Phospholipids E80 | 0.500 |
| Stearylamine | 0.00125 |
| Cross-linked hyaluronic acid | 0.05-0.5 |
| Sodium chloride | 0.450 |
| Boric acid | 0.775 |
| Sodium tetraborate decahydrate | 0.125 |
| Trehalose | 0.30 |
| Distilled water | q.s. to 100 g |

The technical effect of the present invention in the various aspects and in the various embodiments thereof will now be described through the experimentation conducted, by means of which those skilled in the art will be able to appreciate the advantages deriving from its use.

### EXPERIMENTAL PART

The antimicrobial activity of the composition comprising the combination of stearylamine liposomes and hyaluronic acid, or of a salt thereof, both in cross-linked and non-cross-linked form, according to the invention was evaluated by "Challenge test" and the synergistic effect given by the particular association of ingredients of the liposomal formulation was analyzed by comparing the activity of similar compositions which differ in the presence of a component or a technical feature, for example the liposomal formulation, or the cross-linked or non-cross-linked form.

### CHALLENGE TEST 1

Analyzed compositions:
Composition 1: 0.005% w/w stearylamine solution;
Composition 2: 0.005% w/w stearylamine solution with hyaluronic acid sodium salt;
Composition 3: 0.005% w/w stearylamine solution with cross-linked hyaluronic acid sodium salt;
Composition 4: 0.005% w/w stearylamine liposomes;
Composition 5: 0.005% w/w stearylamine solution liposomes with hyaluronic acid sodium salt;
Composition 6: 0.005% w/w stearylamine liposomes with cross-linked hyaluronic acid sodium salt.

Table 1 shows the differences in composition of the solutions tested in the following study.

**Table 1**

| | Composit ion 1 | Composit ion 2 | Composit ion 3 | Composit ion 4 | Composit ion 5 | Compositi on 6 |
|---|---|---|---|---|---|---|
| Phosph olipid s E80 | - | - | - | 0.5% (w/w) | 0.5% (w/w) | 0.5% (w/w) |
| Steary lamine | 0.005% (w/w) | 0.005% (w/w) | 0.005% (w/w) | 0.005% (w/w) | 0.005% (w/w) | 0.005% (w/w) |
| Cross-linked hyalur onic acid | - | - | -0.15 % w/w | - | - | 0.15 %w/w |
| Hyalur onic | - | 0.15 %w/w | - | - | 0.15 %w/w | - |
| acid | | | | | | |
| Sodium chlori de | 0.48% (w/w) | 0.48% (w/w) | 0.48% (w/w) | 0.48% (w/w) | 0.48% (w/w) | 0.48% (w/w) |
| Monoba sic sodium phosph ate dihydr ate | 0.225% (w/w) | 0.225% (w/w) | 0.225% (w/w) | 0.225% (w/w) | 0.225% (w/w) | 0.225% (w/w) |
| Dibasi c sodium phosph ate dihydr ate | 0.685% (w/w) | 0.685% (w/w) | 0.685% (w/w) | 0.685% (w/w) | 0.685% (w/w) | 0.685% (w/w) |
| Trehal ose | 3% (w/w) | 3% (w/w) | 3% (w/w) | 3% (w/w) | 3% (w/w) | 3% (w/w) |
| Distil led water | q.s. to 100 | q.s. to 100 | q.s. to 100 | q.s. to 100 | q.s. to 100 | q.s. to 100 |

| | | | | | | |
|---|---|---|---|---|---|---|
| Strains analyzed: *Pseudomonas aeruginosa* ATCC 9027; *Staphylococcus aureus* ATCC 6538; *Candida albicans* ATCC 10231; *Aspergillus niger* ATCC 16404. | | | | | | |

The counts of the colonies on the plate were performed at T₆ₕ, T₂₄ₕ, T_{7dd}, T_{14dd}, T_{28dd} according to the method of the F.U.I. (Farmacopea Ufficiale Italiana, Italian Official Pharmacopoeia), XII edition. The reference values set by the F.U.I. XII ed. provide an inoculation of bacteria of between 10⁷ - 10⁸ cfu/ml, and an inoculation of fungi and yeasts of between 10⁵ - 10⁶ cfu/ml.

The F.U.I. XII ed. distinguishes between criteria A, which represent the effectiveness that is recommended to be achieved, and criteria B which apply in justified cases where criteria A cannot be met, for example due to an increased risk of undesired reactions. Table 4 shows the fulfillment of the criteria recommended by the F.U.I. XII ed. In order to be able to meet the criteria A, a composition must be capable of providing a reduction of 2 logarithmic units of the concentration of the bacterial inoculum after 6 hours from contact and of three logarithmic units within 24 hours from contact. Criteria B includes the composition capable of providing the reduction of a logarithmic unit within 24 hours and of three logarithmic units within 7 days. As regards fungal strains, the composition meets the criteria A of the Pharmacopoeia when it is capable of providing a reduction of 2 logarithmic units of the initial inoculum concentration after 7 days from contact, the composition meets the criteria B when the reduction amounts to one logarithmic unit after 14 days.

**Table 2 - logarithmic reduction**

| | T₆ₕ | T₂₄ₕ | T_{7dd} | T_{14dd} | T_{28dd} |
|---|---|---|---|---|---|
| Criteria A bacteria | 2 units | 3 units | - | - | NR |
| Criteria B bacteria | - | 1 unit | 3 units | - | No increase, NI |
| Fungi/yeast criteria A | - | - | 2 | - | NI |
| Fungi/yeast criteria B | -- | - | - | 1 | NI |

### Results

### Composition 1 - 0.005% w/w stearylamine solution.

The results relating to the composition 1 comprising 0.005% by weight stearylamine are shown in tables 3 and 4.

**Table 3 - Count of living microorganisms (cfu/ml) as a function of time.**

| ***Pseudomonas aeruginosa* ATCC 9027** | cfu/ml | Log |
|---|---|---|
| Inoculum | 7x10⁷ | 7.8 |
| T₆ₕ | 1x10⁶ | 6.0 |
| T₂₄ₕ | 1x10² | 2.0 |
| T_{7dd} | 1x10² | 2.0 |
| T_{14dd} | 1x10² | 2.0 |
| T_{28dd} | 7x10¹ | 1.8 |

| ***Staphylococcus* aureus ATCC 6538** | cfu/ml | Log |
|---|---|---|
| Inoculum | 1x10⁸ | 8.0 |
| T₆ₕ | 1x10⁷ | 7.0 |
| T₂₄ₕ | 3x10⁴ | 4.5 |
| T_{7dd} | 3x10⁴ | 4.5 |
| T_{14dd} | 3x10⁴ | 4.5 |
| T_{28dd} | 1x10⁴ | 4.0 |

| ***Candida albicans* ATCC 10231** | | |
|---|---|---|
| Inoculum | 2x10⁶ | 6.3 |
| T₆ₕ | 1x10⁶ | 6.0 |
| T₂₄ₕ | 5x10³ | 3.7 |
| T_{7dd} | 5x10³ | 3.7 |
| T_{14dd} | 5x10³ | 3.7 |
| T_{28dd} | 2x10³ | 3.3 |

| ***Aspergillus niger*** ATCC 16404 | | |
|---|---|---|
| Inoculum | 1x10⁶ | 6.0 |
| T₆ₕ | 7x10⁵ | 5.8 |
| T₂₄ₕ | 3x10³ | 3.5 |
| T_{7dd} | 3x10³ | 3.5 |
| T_{14dd} | 3x10³ | 3.5 |
| T_{28dd} | 1x10³ | 3.0 |

**Table 4 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 1.8 | 5.8 | 5.8 | 5.8 | 6.0 |
| ***Staphylococcus** aureus* **ATCC 6538** | 1.0 | 3.5 | 3.5 | 3.5 | 4.0 |
| ***Candida albicans* ATCC 10231** | 0.3 | 2.6 | 2.6 | 2.6 | 3.0 |
| ***Aspergillus niger*** ATCC 16404 | 0.2 | 2.5 | 2.5 | 2.5 | 3.0 |

The composition 1 according to the present invention comprising only stearylamine at a concentration of 0.005% by weight, meets the criteria B recommended by the Italian Official Pharmacopoeia XII ed.; the bacteriostatic activity thereof against *Staphylococcus aureus* (T₂₄ₕ-T_{14dd}) and the gradual decrease in the concentration of the microorganism after 28 days have been demonstrated. Bacteriostatic activity has also been shown against *Pseudomonas aeruginosa* (T₂₄ₕ-T_{14dd}) with slow decrease of the microorganism at 28 days. Finally, the composition 3 tested showed fungistatic action towards *Candida albicans* and *Aspergillus niger* (T₂₄ₕ-T_{14dd}), slow decrease in the concentration of inocula at 28 days. The results obtained in vitro with composition 1 are plotted in figure 1.

### Composition 2 - 0.005% w/w stearylamine solution with hyaluronic acid sodium salt.

The results relating to composition 2 comprising the association of 0.005% by weight stearylamine and non-cross-linked hyaluronic acid sodium salt are shown in tables 5 and 6.

**Table 5 - Count of living microorganisms (cfu/ml) as a function of time.**

| ***Pseudomonas aeruginosa* ATCC 9027** | cfu/ml | Log |
|---|---|---|
| Inoculum | 7x10⁷ | 7.8 |
| T₆ₕ | 3x10⁶ | 6.5 |
| T₂₄ₕ | 8x10⁴ | 4.9 |
| T_{7dd} | 4x10⁴ | 4.6 |
| T_{14dd} | 4x10⁴ | 4.6 |
| T_{28dd} | 1x10³ | 3.0 |

| ***Staphylococcus* aureus ATCC 6538** | cfu/ml | Log |
|---|---|---|
| Inoculum | 1x10⁸ | 8.0 |
| T₆ₕ | 1x10⁷ | 7.0 |
| T₂₄ₕ | 2x10⁴ | 4.3 |
| T_{7dd} | 2x10⁴ | 4.3 |
| T_{14dd} | 2x10⁴ | 4.3 |
| T_{28dd} | 4x10³ | 3.6 |

| ***Candida albicans* ATCC 10231** | | |
|---|---|---|
| Inoculum | 2x10⁶ | 6.3 |
| T₆ₕ | 1x10⁵ | 5.0 |
| T₂₄ₕ | 2x10² | 2.3 |
| T_{7dd} | 7x10¹ | 1.8 |
| T_{14dd} | 7x10¹ | 1.8 |
| T_{28dd} | 4x10¹ | 1.6 |

| ***Aspergillus niger*** ATCC 16404 | | |
|---|---|---|
| Inoculum | 1x10⁶ | 6.0 |
| T₆ₕ | 1x10⁵ | 5.0 |
| T₂₄ₕ | 2x10² | 2.3 |
| T_{7dd} | 1x10² | 2.0 |
| T_{14dd} | 1x10³ | 2.0 |
| T_{28dd} | 3x10¹ | 1.5 |

**Table 6 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 1.3 | 2.9 | 3.2 | 3.2 | 4.8 |
| ***Staphylococcus** aureus* **ATCC 6538** | 1.0 | 3.7 | 3.7 | 3.7 | 4.4 |
| ***Candida albicans* ATCC 10231** | 1.3 | 4.0 | 4.5 | 4.5 | 4.7 |
| ***Aspergillus niger*** ATCC 16404 | 1.0 | 3.7 | 4.0 | 4.0 | 4.5 |

The composition 2 according to the present invention comprising the association of 0.005% by weight stearylamine and non-cross-linked hyaluronic acid sodium salt meets the criteria B recommended by the Italian Official Pharmacopoeia XII ed. The composition was provided with bacteriostatic ability against *Staphylococcus aureus* (T₂₄ₕ-T_{14dd}) with a gradual decrease in the concentration of the microorganism at 28 days, bacteriostatic activity (T_{7dd}-T_{14dd}) and subsequent fair decrease (T_{7dd}-T_{14dd}) thereof is reported against Pseudomonas aeruginosa. Likewise, the analyzed composition has been shown to have fungistatic activity towards *Candida albicans* and *Aspergillus niger* (T_{7dd}-T_{14dd}). The results obtained *in vitro* with composition 2 are plotted in figure 2.

### Composition 3 - 0.005% w/w stearylamine solution with cross-linked hyaluronic acid sodium salt.

The results relating to composition 3 comprising the association of 0.005% by weight stearylamine and cross-linked hyaluronic acid sodium salt are shown in tables 7 and 8.

**Table 7 - Count of living microorganisms (cfu/ml) as a function of time.**

| ***Pseudomonas aeruginosa* ATCC 9027** | cfu/ml | Log |
|---|---|---|
| Inoculum | 7x10⁷ | 7.8 |
| T₆ₕ | 5x10⁷ | 7.7 |
| T₂₄ₕ | 1x10⁴ | 4.0 |
| T_{7dd} | 1x10⁴ | 4.0 |
| T_{14dd} | 1x10⁴ | 4.0 |
| T_{28dd} | 1x10¹ | 1.0 |

| ***Staphylococcus* aureus ATCC 6538** | cfu/ml | Log |
|---|---|---|
| Inoculum | 1x10⁸ | 8.0 |
| T₆ₕ | 1x10⁸ | 8.0 |
| T₂₄ₕ | 6x10⁷ | 7.8 |
| T_{7dd} | 1x10⁴ | 4.0 |
| T_{14dd} | 1x10⁴ | 4.0 |
| T_{28dd} | 17x10¹ | 2.2 |

| ***Candida albicans* ATCC 10231** | | |
|---|---|---|
| Inoculum | 2x10⁶ | 6.3 |
| T₆ₕ | 1x10⁶ | 6.0 |
| T₂₄ₕ | 4x10³ | 3.6 |
| T_{7dd} | 2x10³ | 3.3 |
| T_{14dd} | 25x10¹ | 2.4 |
| T_{28dd} | 2x10¹ | 1.3 |

| ***Aspergillus niger*** ATCC 16404 | | |
|---|---|---|
| Inoculum | 1x10⁶ | 6.0 |
| T₆ₕ | 7x10⁵ | 5.8 |
| T₂₄ₕ | 5x10³ | 3.7 |
| T_{7dd} | 1x10³ | 3.0 |
| T_{14dd} | 1x10² | 2.0 |
| T_{28dd} | 1x10¹ | 1.0 |

**Table 8 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 0.1 | 3.8 | 3.8 | 3.8 | 6.8 |
| ***Staphylococcus aureus* ATCC 6538** | 0.0 | 0.2 | 4.0 | 4.0 | 5.8 |
| ***Candida albicans* ATCC 10231** | 0.3 | 2.7 | 3.0 | 3.9 | 5.0 |
| ***Aspergillus niger*** ATCC 16404 | 0.2 | 2.3 | 3.0 | 4.0 | 5.0 |

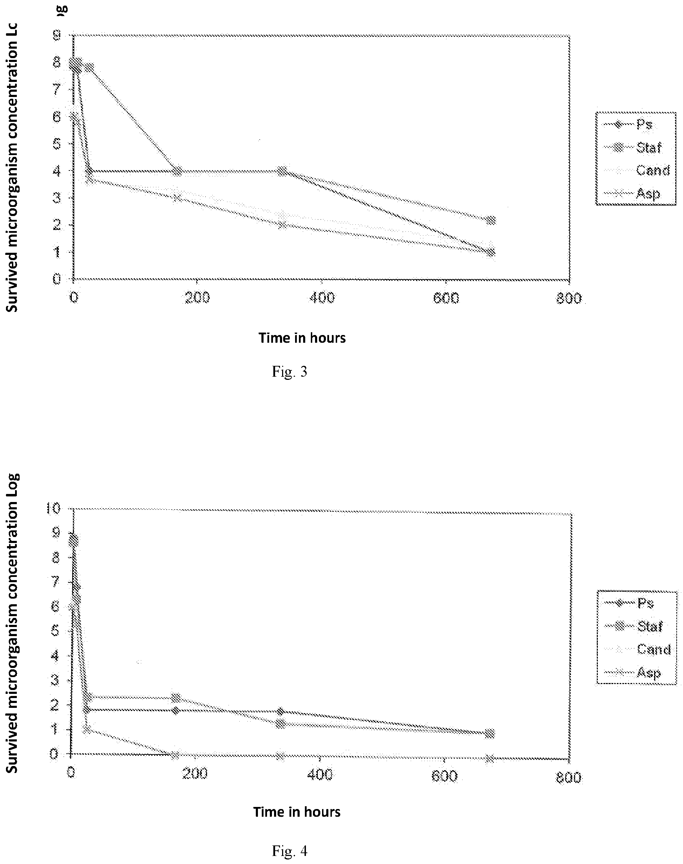

The composition 3 according to the present invention comprising the association of 0.005% by weight stearylamine and cross-linked hyaluronic acid sodium salt meets the criteria B recommended by the Italian Official Pharmacopoeia XII ed.; the bacteriostatic activity against *Pseudomonas aeruginosa* (T₂₄ₕ-T_{14dd}) and *Staphylococcus aureus* (T_{7dd}-T_{14dd}) has been demonstrated, then a good decrease in the concentration of the inocula is observed at 28 days, especially against *Pseudomonas aeruginosa.* Satisfactory activity against *Candida albicans* and *Aspergillus niger.* The results obtained in vitro with composition 3 are plotted in figure 3.

### Composition 4 - 0.005% w/w stearylamine liposomes.

The results relating to the composition 4 comprising liposomes of 0.005% by weight stearylamine are shown in tables 9 and 10.

**Table 9 - Count of living microorganisms (cfu/ml) as a function of time.**

| ***Pseudomonas aeruginosa* ATCC 9027** | cfu/ml | Log |
|---|---|---|
| Inoculum | 6x10⁸ | 8.8 |
| T₆ₕ | 6x10⁶ | 6.8 |
| T₂₄ₕ | 6x10¹ | 1.8 |
| T_{7dd} | 6x10¹ | 1.8 |
| T_{14dd} | 6x10¹ | 1.8 |
| T_{28dd} | 6x10¹ | 1.8 |

| ***Staphylococcus* aureus ATCC 6538** | cfu/ml | Log |
|---|---|---|
| Inoculum | 4x10⁸ | 8.6 |
| T₆ₕ | 2x10⁶ | 6.3 |
| T₂₄ₕ | 20x10¹ | 2.3 |
| T_{7dd} | 20x10¹ | 2.3 |
| T_{14dd} | 2x10¹ | 2.0 |
| T_{28dd} | 1x10¹ | 1.0 |

| ***Candida albicans* ATCC 10231** | | |
|---|---|---|
| Inoculum | 12x10⁵ | 6.1 |
| T₆ₕ | 2x10⁵ | 5.3 |
| T₂₄ₕ | 1x10¹ | 1.0 |
| T_{7dd} | NR | NR |
| T_{14dd} | NR | NR |
| T_{28dd} | NR | NR |

| ***Aspergillus niger* ATCC 16404** | | |
|---|---|---|
| Inoculum | 1x10⁶ | 6.0 |
| T₆ₕ | 2x10⁵ | 5.3 |
| T₂₄ₕ | 1x10¹ | 1.0 |
| T_{7dd} | NR | NR |
| T_{14dd} | NR | NR |
| T_{28dd} | NR | NR |

**Table 10 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 7.0 | 7.0 | 7.0 | 7.8 |
| ***Staphylococcus aureus* ATCC 6538** | 2.3 | 6.3 | 6.3 | 7.3 | 7.6 |
| ***Candida albicans* ATCC 10231** | 0.8 | 5.1 | NR | NR | NR |
| ***Aspergillus niger*** ATCC 16404 | 0.7 | 5.0 | NR | NR | NR |

The composition 4 according to the present invention comprising the liposomal formulation of 0.005% by weight stearylamine meets the criteria A recommended by the Italian Official Pharmacopoeia XII ed.; the bacteriostatic activity against *Staphylococcus aureus* (T₂₄ₕ-T_{7dd}) with a gradual decrease in the concentration of the microorganism at 28 days, and against *Pseudomonas aeruginosa* (T₂₄ₕ-T_{4dd}) with a gradual decrease in the concentration of the microorganism at 28 days, has been demonstrated. Good activity is observed against Candida *albicans* and *Aspergillus niger.* The results obtained in vitro with composition 4 are plotted in figure 4.

### Composition 5 - Liposomes of 0.005% w/w stearylamine solution with hyaluronic acid sodium salt.

The results relating to the composition 5 comprising the liposomal formulation of 0.005% stearylamine in association with the non-cross-linked hyaluronic acid sodium salt are shown in tables 11 and 12.

**Table 11 - Count of living microorganisms (cfu/ml) as a function of time.**

| ***Pseudomonas aeruginosa* ATCC 9027** | cfu/ml | Log |
|---|---|---|
| Inoculum | 3x10⁸ | 8.5 |
| T₆ₕ | 3x10⁶ | 6.5 |
| T₂₄ₕ | 3x10¹ | 1.5 |
| T_{7dd} | 3x10¹ | 1.5 |
| T_{14dd} | 3x10¹ | 1.5 |
| T_{28dd} | 1x10¹ | 1.0 |

| ***Staphylococcus* aureus ATCC 6538** | cfu/ml | Log |
|---|---|---|
| Inoculum | 2x10⁸ | 8.3 |
| T₆ₕ | 1x10⁶ | 6.0 |
| T₂₄ₕ | 1x10⁴ | 4.0 |
| T_{7dd} | 1x10¹ | 1.0 |
| T_{14dd} | 1x10¹ | 1.0 |
| T_{28dd} | 1x10¹ | 1.0 |

| ***Candida albicans* ATCC 10231** | | |
|---|---|---|
| Inoculum | 12x10⁵ | 6.1 |
| T₆ₕ | 12x10⁴ | 5.1 |
| T₂₄ₕ | 1x10¹ | 1.0 |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 1x10⁶ | 6.0 |
| T₆ₕ | 1x10⁵ | 5.0 |
| T₂₄ₕ | 1x10¹ | 1.0 |
| T_{7dd} | NR | NR |
| T_{14dd} | NR | NR |
| T_{28dd} | NR | NR |

**Table 12 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 7.0 | 7.0 | 7.0 | 7.5 |
| ***Staphylococcus aureus* ATCC 6538** | 2.3 | 4.3 | 7.3 | 7.3 | 7.3 |
| ***Candida albicans* ATCC 10231** | 1.0 | 5.1 | NR | NR | NR |
| ***Aspergillus niger*** ATCC 16404 | 1.0 | 5.0 | NR | NR | NR |

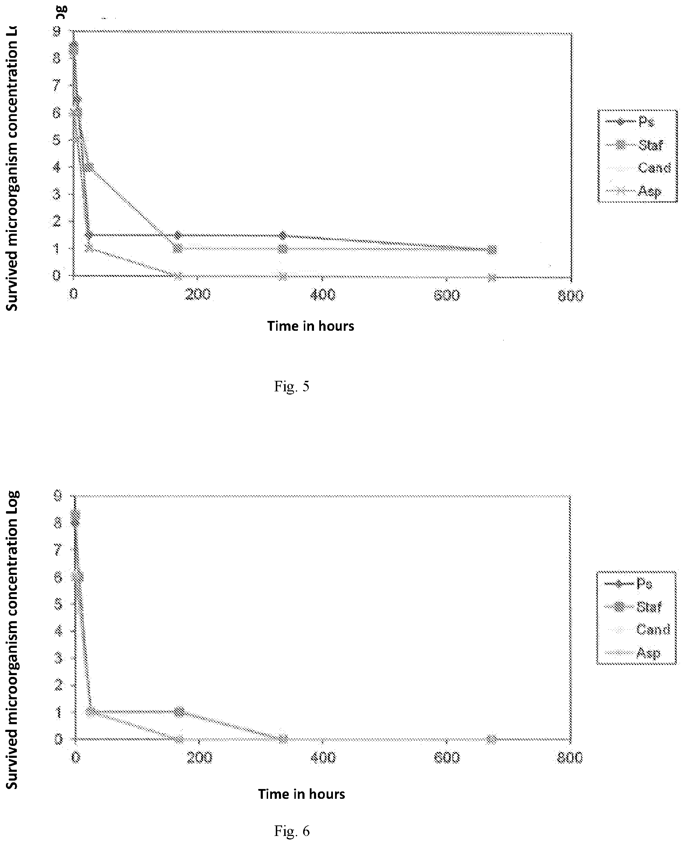

The composition 5 according to the present invention comprising the liposomal formulation of 0.005% by weight stearylamine and non-cross-linked hyaluronic acid sodium salt meets the criteria A recommended by the Italian Official Pharmacopoeia; its bacteriostatic activity against *Staphylococcus aureus* (1_{7dd}-T_{28dd}) and the bacteriostatic activity against *Pseudomonas aeruginosa* (T₂₄ₕ-T_{14dd}) have been demonstrated, with gradual decrease of the microorganism at 28 days, while the microbicidal activity is good against *Candida albicans* and *Aspergillus niger.* The results obtained in vitro with composition 5 are plotted in figure 5.

Composition 6 - Liposomes of 0.005% w/w stearylamine solution with cross-linked hyaluronic acid sodium salt.

The results relating to the composition 5 comprising the liposomal formulation of 0.005% stearylamine in association with the cross-linked hyaluronic acid sodium salt are shown in tables 13 and 14.

**Table 13 - Count of living microorganisms (cfu/ml) as a function of time.**

| ***Pseudomonas aeruginosa* ATCC 9027** | cfu/ml | Log |
|---|---|---|
| Inoculum | 1x10⁸ | 8.0 |
| T₆ₕ | 1x10⁶ | 6.0 |
| T₂₄ₕ | No recovery, NR | NR |
| T_{7dd} | NR | NR |
| T_{14dd} | NR | NR |
| T_{28dd} | NR | NR |

| ***Staphylococcus* aureus ATCC 6538** | cfu/ml | Log |
|---|---|---|
| Inoculum | 2x10⁸ | 8.3 |
| T₆ₕ | 1x10⁶ | 6.0 |
| T₂₄ₕ | 1x10¹ | 1.0 |
| T_{7dd} | 1x10¹ | 1.0 |
| T_{14dd} | NR | NR |
| T_{28dd} | NR | NR |

| ***Candida albicans* ATCC 10231** | | |
|---|---|---|
| Inoculum | 12x10⁵ | 6.1 |
| T₆ₕ | 7x10⁴ | 4.8 |
| T₂₄ₕ | 1x10¹ | 1.0 |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 1x10⁶ | 6.0 |
| T₆ₕ | 1x10⁵ | 5.0 |
| T₂₄ₕ | 1x10¹ | 1.0 |
| T_{7dd} | NR | NR |
| T_{14dd} | NR | NR |
| T_{28dd} | NR | NR |

**Table 14 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 7.0 | NR | NR | NR |
| ***Staphylococcus aureus* ATCC 6538** | 2.3 | 7.3 | 7.3 | 7.3 | NR |
| ***Candida albicans* ATCC 10231** | 1.3 | 5.1 | NR | NR | NR |
| ***Aspergillus niger*** ATCC 16404 | 1.0 | 5.0 | NR | NR | NR |

The composition 6 according to the present invention comprising the liposomal formulation in association with 0.005% stearylamine and cross-linked hyaluronic acid sodium salt meets the criteria A recommended by the Italian Official Pharmacopoeia, its bacteriostatic activity against *Staphylococcus aureus* (T₂₄ₕ-T_{14dd}) has been demonstrated, no recovery of the microorganism has been observed at 28 days while the microbicidal activity is good against *Pseudomonas aeruginosa, Candida albicans* and *Aspergillus niger.* The results obtained in vitro with composition 6 are plotted in figure 6.

The data obtained from the *in vitro* experimentation conducted by means of Challenge test showed first of all the better microbicidal performance of the composition according to the invention comprising the association of a liposomal formulation of 0.005% by weight stearylamine and a sodium salt of hyaluronic acid with respect to the composition having similar formulation but without liposomes (compositions 4, 5, and 6 vs. compositions 1, 2 and 3). The liposomal formulation definitely gives the composition a technical character which improves the final effect thereof. Moreover, another unexpected data is obtained from the experimentation, i.e. the sodium salt of hyaluronic acid in cross-linked form with respect to the non-cross-linked form represents an improving technical feature of the final effect of the composition, both in the liposomal formulation and in the formulation without liposomal structures, as can be seen in the accompanying graphs (composition 2 vs. composition 3, and composition 5 vs. composition 6).

However, stearylamine at a concentration of 0.005% by weight is associated with a certain toxicity which, although not contraindicated in the composition of a product for sanitizing use, for example, to be used for cleaning the house or food or even on the skin of the hands, may not allow hypothesizing the use thereof in a pharmaceutical composition for topical use on the surface of the corneal epithelium.

To this end, the microbicide activity was analyzed using the Challenge test method on the same microbial strains of compositions which according to the present invention comprise the association of a liposomal formulation of stearylamine with hyaluronic acid sodium salt, both in cross-linked form and in non-cross-linked form, at the stearylamine concentration equal to 0.00125 and 0.0025% by weight, respectively.

### CHALLENGE TEST 2

Given the improved performance of the compositions comprising the liposomal formulation, it was decided to focus attention on the latter.

### Analyzed compositions:

Composition 7 0.00125% w/w stearylamine liposomes with cross-linked hyaluronic acid sodium salt;
Composition 8 0.00125% w/w stearylamine solution liposomes with hyaluronic acid sodium salt;
Composition 9 0.00125% w/w stearylamine liposomes;
Composition 10 0.0025% w/w stearylamine liposomes with cross-linked hyaluronic acid sodium salt;
Composition 11 0.0025% w/w stearylamine liposomes with hyaluronic acid sodium salt;
Composition 12: 0.0025% stearylamine liposomes.

Table 15 shows the differences in the composition of the solutions tested in the following study.

**Table 15**

| | Composition 7 | Composition 8 | Composition 9 | Composition 10 | Composition 11 | Composition 12 |
|---|---|---|---|---|---|---|
| Phospholipid s E80 | 0.5% (w/w) | 0.5% (w/w) | 0.5% (w/w) | 0.5% (w/w) | 0.5% (w/w) | 0.5% (w/w) |
| Stearylamine | 0.00125% (w/w) | 0.00125% (w/w) | 0.00125% (w/w) | 0.0025% (w/w) | 0.0025% (w/w) | 0.0025% (w/w) |
| Cross-linked hyaluronic acid | 0.15 % w/w | | - | 0.15 % w/w | | |
| Hyaluronic acid | - | 0.15 % w/w | - | - | 0.15 % w/w | |
| Sodium chloride | 0.48% (w/w) | 0.48% (w/w) | 0.48% (w/w) | 0.48% (w/w) | 0.48% (w/w) | 0.48% (w/w) |
| Monobasic sodium phosphate dihydrate | 0.225% (w/w) | 0.225% (w/w) | 0.225% (w/w) | 0.225% (w/w) | 0.225% (w/w) | 0.225% (w/w) |
| Dibasic sodium phosphate dihydrate | 0.685% (w/w) | 0.685% (w/w) | 0.685% (w/w) | 0.685% (w/w) | 0.685% (w/w) | 0.685% (w/w) |
| Trehalose | 3% (w/w) | 3% (w/w) | 3% (w/w) | 3% (w/w) | 3% (w/w) | 3% (w/w) |
| Distilled water | q.s. to 100 | q.s. to 100 | q.s. to 100 | q.s. to 100 | q.s. to 100 | q.s. to 100 |

### Composition 7 - Liposomes of 0.00125% w/w stearylamine solution with cross-linked hyaluronic acid sodium salt.

The results relating to composition 7 comprising the liposomal formulation of 0.00125% by weight stearylamine and cross-linked hyaluronic acid sodium salt are shown in tables 16 and 17.

**Table 16 - Count of living microorganisms (cfu/ml) as a function of time.**

| ***Pseudomonas aeruginosa* ATCC 9027** | cfu/ml | Log |
|---|---|---|
| Inoculum | 3x10⁸ | 8.5 |
| T₆ₕ | 3x10⁶ | 6.4 |
| T₂₄ₕ | 1x10¹ | 1.0 |
| T_{7dd} | NR | NR |
| T_{14dd} | NR | NR |
| T_{28dd} | NR | NR |

| ***Staphylococcus* aureus ATCC 6538** | cfu/ml | Log |
|---|---|---|
| Inoculum | 9x10⁸ | 8.9 |
| T₆ₕ | 6x10^{s} | 6.8 |
| T₂₄ₕ | 3x10² | 2.5 |
| T_{7dd} | 6x10¹ | 1.8 |
| T_{14dd} | 3x10¹ | 1.5 |
| T_{28dd} | 1x10¹ | 1.0 |

| ***Candida albicans* ATCC 10231** | | |
|---|---|---|
| Inoculum | 1x10⁶ | 6.0 |
| T₆ₕ | 1x10⁴ | 4.0 |
| T₂₄ₕ | 1x10¹ | 1.0 |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 2x10⁶ | 6.3 |
| T₆ₕ | 1x10⁴ | 4.0 |
| T₂₄ₕ | 1x10¹ | 1.0 |
| T_{7dd} | NR | NR |
| T_{14dd} | NR | NR |
| T_{28dd} | NR | NR |

**Table 17 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.1 | 7.5 | NR | NR | NR |
| ***Staphylococcus aureus* ATCC 6538** | 2.1 | 6.4 | 7.1 | 7.4 | 7.9 |
| ***Candida albicans* ATCC 10231** | 2.0 | 5.0 | NR | NR | NR |
| ***Aspergillus niger*** ATCC 16404 | 2.3 | 5.3 | NR | NR | NR |

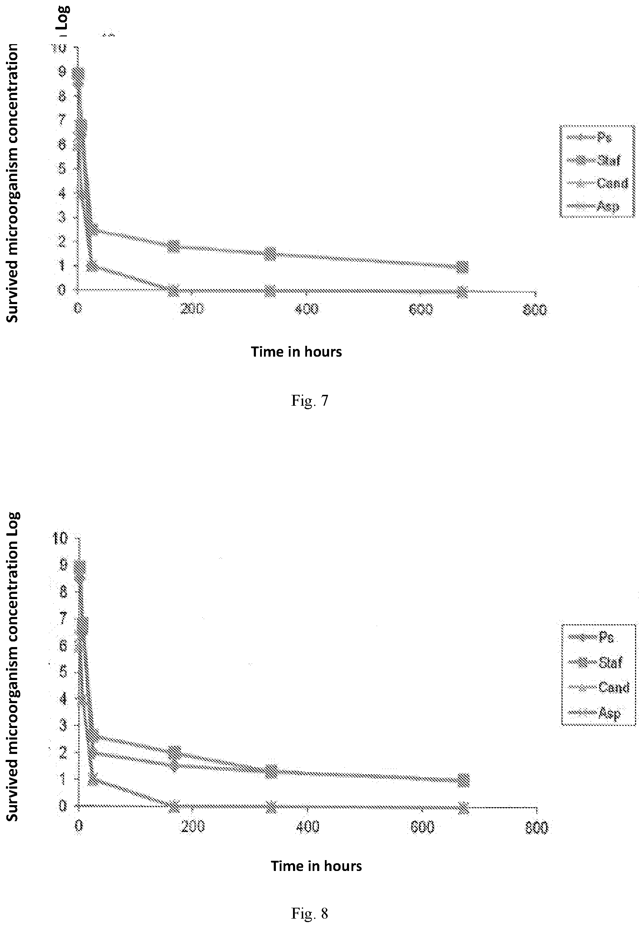

The composition 7 according to the present invention comprising the liposomal formulation of 0.00125% by weight stearylamine and cross-linked hyaluronic acid sodium salt meets the criteria A recommended by the Italian Official Pharmacopoeia: the total reduction of the initial concentration of *Pseudomonas aeruginosa, Candida albicans* and *Aspergillus niger* inocula at T_{7dd} is reported. In relation to the *Staphylococcus aureus* strain, a good reduction in the initial concentration of the inoculum is observed at 7 days and subsequent decrease at 28 days.
The results obtained in vitro with composition 7 are plotted in figure 7.

### Composition 8 - Liposomes of 0.00125% w/w stearylamine solution with hyaluronic acid sodium salt.

The results relating to composition 8 comprising the liposomal formulation of 0.00125% by weight stearylamine and cross-linked hyaluronic acid sodium salt are shown in tables 18 and 19.

**Table 18 - Count of living microorganisms (cfu/ml) as a function of time.**

| ***Pseudomonas aeruginosa* ATCC 9027** | cfu/ml | Log |
|---|---|---|
| Inoculum | 3x10⁸ | 8.5 |
| T₆ₕ | 3x10⁶ | 6.5 |
| T₂₄ₕ | 1x10² | 2.0 |
| T_{7dd} | 3x10¹ | 1.5 |
| T_{14dd} | 2x10¹ | 1.3 |
| T_{28dd} | 1x10¹ | 1.0 |

| ***Staphylococcus* aureus ATCC 6538** | cfu/ml | Log |
|---|---|---|
| Inoculum | 9x10⁸ | 8.9 |
| T₆ₕ | 9x10⁶ | 6.9 |
| T₂₄ₕ | 3x10⁴ | 4.5 |
| T_{7dd} | 9X10² | 2.9 |
| T_{14dd} | 5x10¹ | 1.7 |
| T_{28dd} | 1x10¹ | 1.0 |

| ***Candida albicans* ATCC 10231** | | |
|---|---|---|
| Inoculum | 1x10⁶ | 6.0 |
| T₆ₕ | 1x10⁴ | 4.0 |
| T₂₄ₕ | 1x10¹ | 1.0 |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 2x10⁶ | 6.3 |
| T₆ₕ | 1x10⁴ | 4.0 |
| T₂₄ₕ | 1x10¹ | 1.0 |
| T_{7dd} | NR | NR |
| T_{14dd} | NR | NR |
| T_{28dd} | NR | NR |

**Table 19 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 6.5 | 7.0 | 7.2 | 7.5 |
| ***Staphylococcus aureus* ATCC 6538** | 2.0 | 4.4 | 6.0 | 7.2 | 7.9 |
| ***Candida albicans* ATCC 10231** | 2.0 | 5.0 | NR | NR | NR |
| ***Aspergillus niger*** ATCC 16404 | 2.3 | 5.3 | NR | NR | NR |

The composition 8 according to the present invention comprising the liposomal formulation of 0.00125% by weight stearylamine and non-cross-linked hyaluronic acid sodium salt meets the criteria A recommended by the Italian Official Pharmacopoeia. The results of the trial showed good antifungal activity towards *Candida albicans* and *Aspergillus niger* (no recovery at T_{7dd}), while for the *Staphylococcus aureus* strain (contact time T₂₄ₕ-T_{28dd}) slow decrease of the initial concentration of the inoculum. For *Pseudomonas aeruginosa* (contact times T₂₄ₕ-T_{28dd}), a gradual and slow decrease in the initial concentration of the inoculum is observed, although a good reduction is already evident at shorter contact times (contact times T₆ₕ-T₂₄ₕ).

The results obtained in vitro with composition 8 are plotted in figure 8.

### Composition 9 - 0.00125% w/w stearylamine liposomes.

The results relating to composition 9 comprising the liposomal formulation of 0.00125% by weight stearylamine in association with cross-linked hyaluronic acid sodium salt are shown in tables 20 and 21.

**Table 20 - Count of living microorganisms (cfu/ml) as a function of time.**

| ***Pseudomonas aeruginosa* ATCC 9027** | cfu/ml | Log |
|---|---|---|
| Inoculum | 3x10⁸ | 8.5 |
| T₆ₕ | 3x10⁶ | 6.5 |
| T₂₄ₕ | 1x10² | 2.0 |
| T_{7dd} | 5x10¹ | 1.7 |
| T_{14dd} | 2x10¹ | 1.3 |
| T_{28dd} | 1x10¹ | 1.0 |

| ***Staphylococcus* aureus ATCC 6538** | cfu/ml | Log |
|---|---|---|
| Inoculum | 9x10⁸ | 8.9 |
| T₆ₕ | 6x10⁶ | 6.8 |
| T₂₄ₕ | 3x10² | 2.5 |
| T_{7dd} | 1x10² | 2.0 |
| T_{14dd} | 3x10¹ | 1.5 |
| T_{28dd} | 1x10¹ | 1.0 |

| ***Candida albicans* ATCC 10231** | | |
|---|---|---|
| Inoculum | 1x10⁶ | 6.0 |
| T₆ₕ | 1x10⁴ | 4.0 |
| T₂₄ₕ | 1x10² | 2.0 |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 2x10⁶ | 6.3 |
| T₆ₕ | 1x10⁴ | 4.0 |
| T₂₄ₕ | 1x10² | 2.0 |
| T_{7dd} | NR | NR |
| T_{14dd} | NR | NR |
| T_{28dd} | NR | NR |

**Table 21 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 6.5 | 6.8 | 7.2 | 7.5 |
| ***Staphylococcus aureus* ATCC 6538** | 2.1 | 6.4 | 6.9 | 7.4 | 7.9 |
| ***Candida albicans* ATCC 10231** | 2.0 | 4.0 | NR | NR | NR |
| ***Aspergillus niger*** ATCC 16404 | 2.3 | 4.3 | NR | NR | NR |

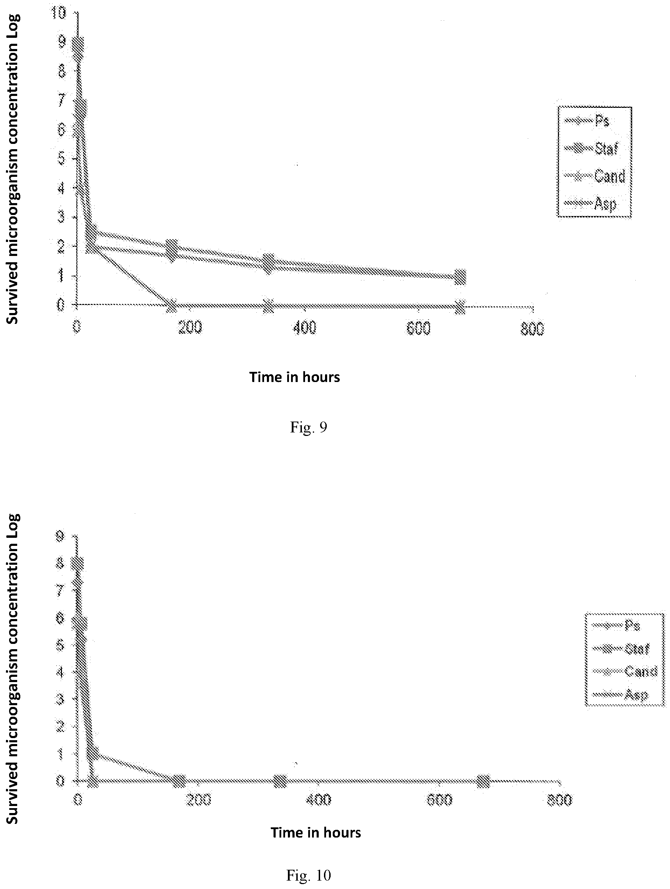

The composition 9 according to the present invention comprising the liposomal formulation of 0.00125% by weight stearylamine in association with non-cross-linked hyaluronic acid sodium salt meets the criteria A recommended by the Italian Official Pharmacopoeia. The results of the trial showed good antifungal activity towards Candida albicans and Aspergillus niger (no recovery at T7dd), while for the *Staphylococcus aureus* and *Pseudomonas aeruginosa* strains, gradual decreases are observed in the initial concentrations of inocula at 28 days (contact times T_{7dd}-T_{28dd}), although a good reduction is already observed at shorter contact times (T₆ₕ-T₂₄ₕ). The results are shown in the graph in figure 9.

### Composition 10 - Liposomes of 0.0025% w/w stearylamine solution with cross-linked hyaluronic acid sodium salt.

The results relating to composition 10 comprising the liposomal formulation of 0.0025% by weight stearylamine and cross-linked hyaluronic acid sodium salt are shown in tables 22 and 23.

**Table 22 - Count of living microorganisms (cfu/ml) as a function of time.**

| ***Pseudomonas aeruginosa* ATCC 9027** | cfu/ml | Log |
|---|---|---|
| Inoculum | 6x10⁷ | 7.8 |
| T₆ₕ | 6x10⁷ | 7.8 |
| T₂₄ₕ | 6x10⁵ | 5.8 |
| T_{7dd} | 19x10⁴ | 5.3 |
| T_{14dd} | 7x10⁴ | 4.8 |
| T_{28dd} | 19x10³ | 4.3 |

| ***Staphylococcus* aureus ATCC 6538** | cfu/ml | Log |
|---|---|---|
| Inoculum | 1x10⁸ | 8.0 |
| T₆ₕ | 1x10⁸ | 8.0 |
| T₂₄ₕ | 1x10⁸ | 8.0 |
| T_{7dd} | 1x10⁶ | 6.0 |
| T_{14dd} | 3x10⁴ | 4.5 |
| T_{28dd} | 1x10⁴ | 4.0 |

| ***Candida albicans* ATCC 10231** | | |
|---|---|---|
| Inoculum | 1x10⁶ | 6.0 |
| T₆ₕ | 1x10⁶ | 6.0 |
| T₂₄ₕ | 1x10⁶ | 6.0 |
| T_{7dd} | 3x10³ | 3.5 |
| T_{14dd} | 1x10³ | 3.0 |
| T_{28dd} | 1x10³ | 3.0 |

| ***Aspergillus niger*** ATCC 16404 | | |
|---|---|---|
| Inoculum | 7x10⁵ | 5.8 |
| T₆ₕ | 7x10⁵ | 5.8 |
| T₂₄ₕ | 7x10⁵ | 5. 8 |
| T_{7dd} | 6x10² | 2.8 |
| T_{14dd} | 6x10² | 2.8 |
| T_{28dd} | 6x10² | 2.8 |

**Table 23 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 0.0 | 2.0 | 2.5 | 3.0 | 3.5 |
| ***Staphylococcus aureus* ATCC 6538** | 0.0 | 0.0 | 2.0 | 3.5 | 4.0 |
| ***Candida albicans* ATCC 10231** | 0.0 | 0.0 | 2.5 | 3.0 | 3.0 |
| ***Aspergillus niger*** ATCC 16404 | 0.0 | 0.0 | 3.0 | 3.0 | 3.0 |

The composition 10 according to the present invention comprising the liposomal formulation of 0.0025% by weight stearylamine in association with non-cross-linked hyaluronic acid sodium salt does not meet the criteria recommended by the Italian Official Pharmacopoeia. XII ed. since criteria A are met only for fungi and yeasts, and for *Pseudomonas aeruginosa* only criteria B are met. Indeed, the results of the Challenge test showed no reduction in the initial concentration of the *Pseudomonas aeruginosa* inoculum at 6 hours and a slow reduction up to 28 days, although a reduction of 2 logarithmic units was evident at the 24-hour contact time. For *Staphylococcus aureus,* there is no reduction in the initial concentration of the inoculum at 24 hours and a gradual reduction up to 28 days, although a reduction of 1.5 logarithmic units is recorded at contact times T_{7dd}-T_{14dd}.
For *Candida albicans* and *Aspergillus niger,* no reduction of the initial concentrations of the inocula at 24 hours and fungistatic activity in the contact time T_{7dd}-T_{28dd} has been highlighted, although a logarithmic reduction appears at T_{7dd} according to the specifications A of the Pharmacopoeia.

The results obtained in vitro with composition 10 are plotted in figure 10.

### Composition 11 - Liposomes of 0.0025% w/w stearylamine solution with hyaluronic acid sodium salt.

The results relating to composition 11 comprising the liposomal formulation of 0.0025% stearylamine and non-cross-linked hyaluronic acid sodium salt are shown in tables 24 and 25.

**Table 24 - Count of living microorganisms (cfu/ml) as a function of time.**

| ***Pseudomonas aeruginosa* ATCC 9027** | cfu/ml | Log |
|---|---|---|
| Inoculum | 1x10⁸ | 8.0 |
| T₆ₕ | 1x10⁶ | 6.0 |
| T₂₄ₕ | 1x10¹ | 1.0 |
| T_{7dd} | NR | NR |
| T_{14dd} | NR | NR |
| T_{28dd} | NR | NR |

| ***Staphylococcus* aureus ATCC 6538** | cfu/ml | Log |
|---|---|---|
| Inoculum | 9x10⁸ | 8.9 |
| T₆ₕ | 6x10⁶ | 6.8 |
| T₂₄ₕ | 4x10² | 2.6 |
| T_{7dd} | 1x10² | 2.0 |
| T_{14dd} | 2x10¹ | 1.3 |
| T_{28dd} | 1x10¹ | 1.0 |

| ***Candida albicans* ATCC 10231** | | |
|---|---|---|
| Inoculum | 1x10⁶ | 6.0 |
| T₆ₕ | 1x10⁴ | 4.0 |
| T₂₄ₕ | NR | NR |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 2x10⁶ | 6.3 |
| T₆ₕ | 6x10³ | 3.8 |
| T₂₄ₕ | NR | NR |
| T_{7dd} | NR | NR |
| T_{14dd} | NR | NR |
| T_{28dd} | NR | NR |

**Table 25 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 2.0 | 7.0 | NR | NR | NR |
| ***Staphylococcus aureus* ATCC 6538** | 2.1 | 6.3 | 6.9 | 7.6 | 7.9 |
| ***Candida albicans* ATCC 10231** | 2.0 | NR | NR | NR | NR |
| ***Aspergillus niger*** ATCC 16404 | 0.0 | NR | NR | NR | NR |

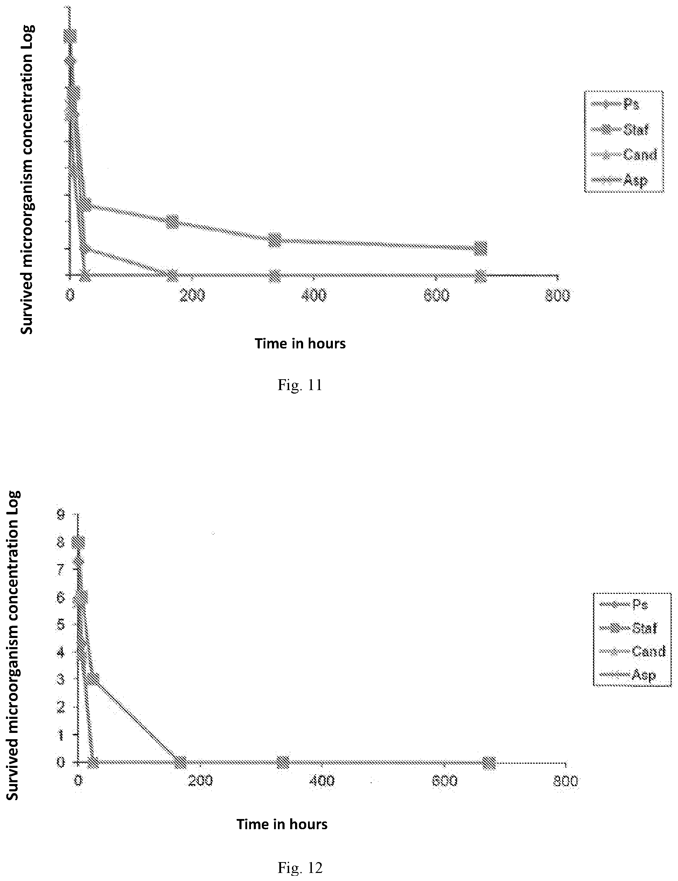

The composition 11 according to the present invention comprising the liposomal formulation of 0.0025% by weight stearylamine in association with non-cross-linked hyaluronic acid sodium salt meets the criteria A recommended by the Italian Official Pharmacopoeia. XII ed. since good antibacterial activity against *Pseudomonas aeruginosa* (no recovery at 7 days) and antifungal activity against *Candida albicans* and *Aspergillus niger* was observed, no recovery at 24 hours. For *Staphylococcus aureus* strain, a good reduction in the initial concentration of the inoculum is observed at 7 days and subsequent gradual decrease at 28 days. The results obtained are shown in the graph in figure 11.

### Composition 12 - Stearylamine liposomes of 0.0025% w/w.

The results relating to the composition 12 comprising 0.00125% stearylamine liposomes are shown in tables 26 and 27.

**Table 26 - Count of living microorganisms (cfu/ml) as a function of time.**

| ***Pseudomonas aeruginosa* ATCC 9027** | cfu/ml | Log |
|---|---|---|
| Inoculum | 2x10⁷ | 7.3 |
| T₆ₕ | 19x10⁶³ | 4.3 |
| T₂₄ₕ | NR | NR |
| T_{7dd} | NR | NR |
| T_{14dd} | NR | NR |
| T_{28dd} | NR | NR |

| ***Staphylococcus* aureus ATCC 6538** | cfu/ml | Log |
|---|---|---|
| Inoculum | 1x10⁸ | 8.0 |
| T₆ₕ | 1x10⁶ | 6.0 |
| T₂₄ₕ | 1x10³ | 3.0 |
| T_{7dd} | NR | NR |
| T_{14dd} | NR | NR |
| T_{28dd} | NR | NR |

| ***Candida albicans* ATCC 10231** | | |
|---|---|---|
| Inoculum | 1x10⁶ | 6.0 |
| T₆ₕ | 1x10⁴ | 4.0 |
| T₂₄ₕ | NR | NR |
| T_{7dd} | NR | NR |
| T_{14dd} | NR | NR |
| T_{28dd} | NR | NR |

| ***Aspergillus niger*** ATCC 16404 | | |
|---|---|---|
| Inoculum | 7x10⁵ | 5.8 |
| T₆ₕ | 6x10³ | 3.8 |
| T₂₄ₕ | NR | NR |
| T_{7dd} | NR | NR |
| T_{14dd} | NR | NR |
| T_{28dd} | NR | NR |

**Table 27 - Logarithmic reduction in the number of microorganisms as a function of time.**

| | T₆ₕ | T₂₄ₕ | T_{7dd} | T_{14dd} | T_{28dd} |
|---|---|---|---|---|---|
| ***Pseudomonas aeruginosa* ATCC 9027** | 3.0 | NR | NR | NR | NR |
| ***Staphylococcus aureus* ATCC 6538** | 2.0 | 5.0 | NR | NR | NR |
| ***Candida albicans* ATCC 10231** | 2.0 | NR | NR | NR | NR |
| ***Aspergillus niger*** ATCC 16404 | 2.0 | NR | NR | NR | NR |

The composition 12 according to the present invention comprising the liposomal formulation of 0.0025% stearylamine meets the criteria A recommended by the Italian Official Pharmacopoeia. The results of the trial showed a good antibacterial and antifungal activity against all the strains tested in the test. In particular, the total reduction of the initial concentration of the *Pseudomonas aeruginosa, Candida albicans* and *Aspergillus niger* inocula is reported at T₂₄ₕ and at T_{7dd} against the *Staphylococcus aureus* strain.

The results obtained in vitro with composition 12 are plotted in figure 12.

From the results of this second Challenge test conducted on liposomal formulations according to the present invention, the best performance of the compositions with the association of stearylamine and hyaluronic acid sodium salt was confirmed, in particular those in which this salt is in cross-linked form. Furthermore, by decreasing the concentration of stearylamine, the problem of the possible toxicity of the composition is eliminated, thus making it suitable for countering microbial infections even in a topical use on delicate epithelia of the human and animal body, such as the corneal epithelium, thus providing an ophthalmic composition with proven antimicrobial activity.

## Claims

1. An association of stearylamine liposomes with hyaluronic acid, or a sodium salt thereof, wherein phospholipids and stearylamine are at various molar ratio in liposomes, with phospholipids equal to 0.5% by weight and stearylamine ranging between 0.001 and 0.010% by weight, for use in the treatment of microbial infections caused by bacteria, fungi and *Candida albicans.*

2. An association for use according to claim 1, wherein stearylamine ranges between 0.0012 and 0.005% by weight, and hyaluronic acid, or the sodium salt thereof, ranges between 0.05 and 0.5% by weight.

3. An association for use according to claim 2, wherein stearylamine is equal to 0.025% by weight and hyaluronic acid or the sodium salt thereof ranges between 0.1 and 0.3 by weight, preferably 0.15% by weight.

4. An association for use according to claims 1, 2 and 3, wherein hyaluronic acid is in cross-linked form.

5. An association for use according to claim 1, wherein the infections are ophthalmic infections.

6. An association of stearylamine liposomes with hyaluronic acid, or a sodium salt thereof, wherein phospholipids and stearylamine are at various molar ratio in liposomes, with phospholipids equal to 0.5% by weight and stearylamine ranging between 0.001 and 0.010% by weight, for use in a treatment method for cleaning and disinfecting surfaces in a domestic environment and/or food service areas, hospitals and the like.

7. An association of stearylamine liposomes with hyaluronic acid, or a sodium salt thereof, wherein phospholipids and stearylamine are at various molar ratio in liposomes, with phospholipids equal to 0.5% by weight and stearylamine ranging between 0.001 and 0.010% by weight, for use in a method of treating the surface of a human and animal body and in personal hygiene.

8. An association for use according to claim 6, wherein stearylamine ranges between 0.0012 and 0.005% by weight, and hyaluronic acid, or the sodium salt thereof, ranges between 0.05 and 0.5% by weight.

9. An association for use according to claim 6, wherein hyaluronic acid is in a cross-linked form.

10. Association for use as in claim 7 of stearylamine liposomes with hyaluronic acid, or a sodium salt thereof, wherein phospholipids and stearylamine are at various molar ratios in liposomes, where hyaluronic acid is in cross-linked form.

11. A composition comprising an association of stearylamine liposomes with hyaluronic acid, or a sodium salt thereof, wherein phospholipids and stearylamine are at various molar ratio in liposomes, with phospholipids equal to 0.5% by weight and stearylamine ranging between 0.001 and 0.010% by weight, and pharmaceutically accepted excipients.

12. A composition according to claim 11, wherein stearylamine ranges between 0.0012 and 0.005% by weight, and hyaluronic acid, or the sodium salt thereof, ranges between 0.05 and 0.5% by weight.

13. A composition according to claim 12, wherein stearylamine is 0.025% by weight and hyaluronic acid, or the sodium salt thereof, ranges between 0.1 and 0.3% by weight.

14. A composition according to claim 13, wherein stearylamine is 0.025% by weight and hyaluronic acid, or the sodium salt thereof, is equal to 0.15% by weight.

15. A composition according to any one of claims 11-14, wherein hyaluronic acid is in cross-linked form.

16. A composition according to claims 11 to 15 further comprising active ingredients, buffering agents, preservatives, diluents, carriers and wetting agents.

17. A composition according to claim 16, wherein the buffering agents are the monobasic/dibasic sodium phosphate system, the borate system and the citrate buffer system.

18. A composition according to any one of claims 11 to 17 having the following formulation, where the amount of each ingredient is expressed in percentage by weight:
| | |
|---|---|
| Phospholipids E80 | 0.500% w/w |
| Stearylamine | 0.0025% w/w |
| Cross-linked hyaluronic acid | 0.15% w/w |
| Sodium chloride | 0.480% w/w |
| Monobasic sodium phosphate dihydrate | 0.225% w/w |
| Dibasic sodium phosphate dihydrate | 0.685% w/w |
| Trehalose | 0.30% w/w |
| Distilled water | q.s. to 100 g. |

19. A composition according to any one of claims 11 to 17 having the following formulation, where the amount of each ingredient is expressed in percentage by weight:
| | |
|---|---|
| Phospholipids E80 | 0.500% w/w |
| Stearylamine | 0.0025% w/w |
| Cross-linked hyaluronic acid | 0.15% w/w |
| Sodium chloride | 0.480% w/w |
| Boric acid | 0.775% w/w |
| Sodium tetraborate decahydrate | 0.125% w/w |
| Trehalose | 0.30% w/w |
| Distilled water | q.s. to 100 g. |

20. A composition according to any one of claims 11 to 19 sterilized by filtration at 0.25 microns of porosity.

21. A composition according to any one of claims 11 to 20 for medical use.

22. A composition according to any one of claims 11 to 20, for use in the treatment of microbial infections caused by bacteria, fungi and *Candida albicans.*

23. A composition for use according to claim 22, wherein the infections are ophthalmic infections.

24. A composition according to claims 11 to 19 for use in a treatment for the disinfection of the surface of the human and animal body, and for personal hygiene.

25. A composition according to claims 11 to 19 for use in cleaning and disinfecting surfaces in a domestic environment and/or food service areas, hospitals.

## Patentansprüche

1. Assoziation von Stearylamin-Liposomen mit Hyaluronsäure oder einem Natriumsalz davon, wobei Phospholipide und Stearylamin in verschiedenen molaren Verhältnissen in Liposomen vorliegen, mit Phospholipiden gleich 0,5 Gew.-% und Stearylamin im Bereich zwischen 0,001 und 0,010 Gew.-%, zur Verwendung bei der Behandlung von mikrobiellen Infektionen, verursacht durch Bakterien, Pilze und Candida albicans.

2. Assoziation zur Verwendung nach Anspruch 1, wobei Stearylamin im Bereich zwischen 0,0012 und 0,005 Gew.-% liegt und Hyaluronsäure oder das Natriumsalz davon im Bereich zwischen 0,05 und 0,5 Gew.-% liegt.

3. Assoziation zur Verwendung nach Anspruch 2, wobei Stearylamin gleich 0,025 Gew.-% ist und Hyaluronsäure oder das Natriumsalz davon im Bereich zwischen 0,1 und 0,3 Gew.-% liegt, vorzugsweise 0,15 Gew.-%.

4. Assoziation zur Verwendung nach den Ansprüchen 1, 2 und 3, wobei Hyaluronsäure in vernetzter Form vorliegt.

5. Assoziation zur Verwendung nach Anspruch 1, wobei die Infektionen ophthalmische Infektionen sind.

6. Assoziation von Stearylamin-Liposomen mit Hyaluronsäure oder einem Natriumsalz davon, wobei Phospholipide und Stearylamin in verschiedenen molaren Verhältnissen in Liposomen vorliegen, mit Phospholipiden gleich 0,5 Gew.-% und Stearylamin im Bereich zwischen 0,001 und 0,010 Gew.-%, zur Verwendung in einem Behandlungsverfahren zur Reinigung und Desinfektion von Oberflächen in einer häuslichen Umgebung und/oder in Bereichen der Lebensmittelversorgung, in Krankenhäusern und dergleichen.

7. Assoziation von Stearylamin-Liposomen mit Hyaluronsäure oder einem Natriumsalz davon, wobei Phospholipide und Stearylamin in verschiedenen molaren Verhältnissen in den Liposomen vorliegen, mit Phospholipiden gleich 0,5 Gew.-% und Stearylamin im Bereich zwischen 0,001 und 0,010 Gew.-%, zur Verwendung in einem Verfahren zur Behandlung der Oberfläche eines menschlichen und tierischen Körpers und in der persönlichen Hygiene.

8. Assoziation zur Verwendung nach Anspruch 6, wobei Stearylamin im Bereich zwischen 0,0012 und 0,005 Gew.-% und Hyaluronsäure oder das Natriumsalz davon im Bereich zwischen 0,05 und 0,5 Gew.-% vorliegt.

9. Assoziation zur Verwendung nach Anspruch 6, wobei Hyaluronsäure in einer vernetzten Form vorliegt.

10. Assoziation zur Verwendung nach Anspruch 7 von Stearylamin-Liposomen mit Hyaluronsäure oder einem Natriumsalz davon, wobei Phospholipide und Stearylamin in verschiedenen molaren Verhältnissen in den Liposomen vorliegen, wobei Hyaluronsäure in vernetzter Form vorliegt.

11. Zusammensetzung, umfassend eine Assoziation von Stearylamin-Liposomen mit Hyaluronsäure oder einem Natriumsalz davon, wobei Phospholipide und Stearylamin in verschiedenen molaren Verhältnissen in Liposomen vorliegen, mit Phospholipiden gleich 0,5 Gew.-% und Stearylamin im Bereich zwischen 0,001 und 0,010 Gew.-%, sowie pharmazeutisch akzeptierte Hilfsstoffe.

12. Zusammensetzung nach Anspruch 11, wobei Stearylamin im Bereich zwischen 0,0012 und 0,005 Gew.-% und Hyaluronsäure oder das Natriumsalz davon im Bereich zwischen 0,05 und 0,5 Gew.-% vorliegt.

13. Zusammensetzung nach Anspruch 12, wobei Stearylamin 0,025 Gew.-% und Hyaluronsäure oder das Natriumsalz davon im Bereich zwischen 0,1 und 0,3 Gew.-% vorliegt.

14. Zusammensetzung nach Anspruch 13, wobei Stearylamin 0,025 Gew.-% und Hyaluronsäure oder das Natriumsalz davon gleich 0,15 Gew.-% ist.

15. Zusammensetzung nach einem der Ansprüche 11-14, wobei Hyaluronsäure in vernetzter Form vorliegt.

16. Zusammensetzung nach einem der Ansprüche 11 bis 15, ferner umfassend Wirkstoffe, Puffermittel, Konservierungsmittel, Verdünnungsmittel, Träger und Benetzungsmittel.

17. Zusammensetzung nach Anspruch 16, wobei die Puffermittel das einbasische/zweibasische Natriumphosphatsystem, das Boratsystem und das Citratpuffersystem sind.

18. Zusammensetzung nach einem der Ansprüche 11 bis 17, die die folgende Formulierung aufweist, wobei die Menge jedes Inhaltsstoffs in Gewichtsprozent angegeben ist:
| | |
|---|---|
| Phospholipide E80 | 0,500% w/w |
| Stearylamin | 0,0025% w/w |
| Vernetzte Hyaluronsäure | 0,15 % w/w |
| Natriumchlorid | 0,480% w/w |
| Monobasisches Natriumphosphat-Dihydrat | 0,225% w/w |
| Zweibasisches Natriumphosphat-Dihydrat | 0,685% w/w |
| Trehalose | 0,30% w/w |
| Destilliertes Wasser | q.s. bis 100 g. |

19. Zusammensetzung nach einem der Ansprüche 11 bis 17, die die folgende Formulierung aufweist, wobei die Menge jedes Inhaltsstoffs in Gewichtsprozent angegeben ist:
| | |
|---|---|
| Phospholipide E80 | 0,500% w/w |
| Stearylamin | 0,0025% w/w |
| Vernetzte Hyaluronsäure | 0,15 % w/w |
| Natriumchlorid | 0,480 % w/w |
| Borsäure | 0,775% w/w |
| Natriumtetraborat-Decahydrat | 0,125% w/w |
| Trehalose | 0,30% w/w |
| Destilliertes Wasser | q.s. bis 100 g. |

20. Zusammensetzung nach einem der Ansprüche 11 bis 19, sterilisiert durch Filtration mit einer Porosität von 0,25 Mikrometern.

21. Zusammensetzung nach einem der Ansprüche 11 bis 20 zur medizinischen Verwendung.

22. Zusammensetzung nach einem der Ansprüche 11 bis 20 zur Verwendung bei der Behandlung von mikrobiellen Infektionen, verursacht durch Bakterien, Pilze und Candida albicans.

23. Zusammensetzung zur Verwendung nach Anspruch 22, wobei die Infektionen ophthalmische Infektionen sind.

24. Zusammensetzung nach einem der Ansprüche 11 bis 19 zur Verwendung bei einer Behandlung zur Desinfektion der Oberfläche des menschlichen und tierischen Körpers und zur persönlichen Hygiene.

25. Zusammensetzung nach einem der Ansprüche 11 bis 19 zur Verwendung bei der Reinigung und Desinfektion von Oberflächen im häuslichen Umfeld und/oder in der Gastronomie, in Krankenhäusern.

## Revendications

1. Association de liposomes de stéarylamine et d'acide hyaluronique, ou d'un sel sodique de celui-ci, dans laquelle des phospholipides et de la stéarylamine sont présents à divers rapports molaires dans des liposomes, les phospholipides étant égaux à 0,5 % en poids et la stéarylamine étant dans une plage entre 0,001 et 0,010 % en poids, pour une utilisation dans le traitement d'infections microbiennes provoquées par des bactéries, des champignons et *Candida albicans.*

2. Association pour une utilisation selon la revendication 1, dans laquelle la stéarylamine est dans une plage entre 0,0012 et 0,005 % en poids, et l'acide hyaluronique, ou le sel sodique de celui-ci, est dans une plage entre 0,05 et 0,5 % en poids.

3. Association pour une utilisation selon la revendication 2, dans laquelle la stéarylamine est égale à 0,025 % en poids et l'acide hyaluronique ou le sel sodique de celui-ci est dans une plage entre 0,1 et 0,3 % en poids, de préférence 0,15 % en poids.

4. Association pour une utilisation selon les revendications 1, 2 et 3, dans laquelle l'acide hyaluronique est sous forme réticulée.

5. Association pour une utilisation selon la revendication 1, dans laquelle les infections sont des infections ophtalmiques.

6. Association de liposomes de stéarylamine et d'acide hyaluronique, ou d'un sel sodique de celui-ci, dans laquelle des phospholipides et de la stéarylamine sont présents à divers rapports molaires dans des liposomes, les phospholipides étant égaux à 0,5 % en poids et la stéarylamine étant dans une plage entre 0,001 et 0,010 % en poids, pour une utilisation dans un procédé de traitement de nettoyage et de désinfection de surfaces dans un environnement domestique et/ou dans des zones de service alimentaire, des hôpitaux et similaires.

7. Association de liposomes de stéarylamine et d'acide hyaluronique, ou d'un sel sodique de celui-ci, dans laquelle des phospholipides et de la stéarylamine sont présents à divers rapports molaires dans des liposomes, les phospholipides étant égaux à 0,5 % en poids et la stéarylamine étant dans une plage entre 0,001 et 0,010 % en poids, pour une utilisation dans un procédé de traitement de la surface d'un corps humain ou animal et en hygiène personnelle.

8. Association pour une utilisation selon la revendication 6, dans laquelle la stéarylamine est dans une plage entre 0,0012 et 0,005 % en poids, et l'acide hyaluronique, ou le sel sodique de celui-ci, est dans une plage entre 0,05 et 0,5 % en poids.

9. Association pour une utilisation selon la revendication 6, dans laquelle l'acide hyaluronique est sous forme réticulée.

10. Association pour une utilisation selon la revendication 7 de liposomes de stéarylamine et d'acide hyaluronique, ou d'un sel sodique de celui-ci, dans laquelle des phospholipides et de la stéarylamine sont présents à divers rapports molaires dans des liposomes, où l'acide hyaluronique est sous forme réticulée.

11. Composition comprenant une association de liposomes de stéarylamine et d'acide hyaluronique, ou d'un sel sodique de celui-ci, dans laquelle des phospholipides et de la stéarylamine sont présents à divers rapports molaires dans des liposomes, les phospholipides étant égaux à 0,5 % en poids et la stéarylamine étant dans une plage entre 0,001 et 0,010 % en poids, et des excipients pharmaceutiquement acceptables.

12. Composition selon la revendication 11, dans laquelle la stéarylamine est dans une plage entre 0,0012 et 0,005 % en poids, et l'acide hyaluronique, ou le sel sodique de celui-ci, est dans une plage entre 0,05 et 0,5 % en poids.

13. Composition selon la revendication 12, dans laquelle la stéarylamine est à 0,025 % en poids et l'acide hyaluronique, ou le sel sodique de celui-ci, est dans une plage entre 0,1 et 0,3 % en poids.

14. Composition selon la revendication 13, dans laquelle la stéarylamine est à 0,025 % en poids et l'acide hyaluronique, ou le sel sodique de celui-ci, est égal à 0,15 % en poids.

15. Composition selon l'une quelconque des revendications 11 à 14, dans laquelle l'acide hyaluronique est sous forme réticulée.

16. Composition selon les revendications 11 à 15, comprenant en outre des ingrédients actifs, des agents tampons, des conservateurs, des diluants, des supports et des agents mouillants.

17. Composition selon la revendication 16, dans laquelle les agents tampons sont le système phosphate sodique monobasique/dibasique, le système borate et le système tampon citrate.

18. Composition selon l'une quelconque des revendications 11 à 17 ayant la formulation suivante, où la quantité de chaque ingrédient est exprimée en pourcentage en poids :
| | |
|---|---|
| Phospholipides E80 | 0,500 % p/p |
| Stéarylamine | 0,0025 % p/p |
| Acide hyaluronique réticulé | 0,15 % p/p |
| Chlorure de sodium | 0,480 % p/p |
| Phosphate sodique dihydraté monobasique | 0,225 % p/p |
| Phosphate sodique dihydraté dibasique | 0,685 % p/p |
| Tréhalose | 0,30 % p/p |
| Eau distillée | q.s. à 100 g |

19. Composition selon l'une quelconque des revendications 11 à 17 ayant la formulation suivante, où la quantité de chaque ingrédient est exprimée en pourcentage en poids :
| | |
|---|---|
| Phospholipides E80 | 0,500 % p/p |
| Stéarylamine | 0,0025 % p/p |
| Acide hyaluronique réticulé | 0,15 % p/p |
| Chlorure de sodium | 0,480 % p/p |
| Acide borique | 0,775 % p/p |
| Tétraborate sodique décahydraté | 0,125 % p/p |
| Tréhalose | 0,30 % p/p |
| Eau distillée | q.s. à 100 g |

20. Composition selon l'une quelconque des revendications 11 à 19 stérilisée par filtration à 0,25 micron de porosité.

21. Composition selon l'une quelconque des revendications 11 à 20 pour une utilisation médicale.

22. Composition selon l'une quelconque des revendications 11 à 20, pour une utilisation dans le traitement d'infections microbiennes provoquées par des bactéries, des champignons et *Candida albicans.*

23. Composition pour une utilisation selon la revendication 22, dans laquelle les infections sont des infections ophtalmiques.

24. Composition selon les revendications 11 à 19 pour une utilisation dans un traitement pour la désinfection de la surface du corps humain et animal, et pour l'hygiène personnelle.

25. Composition selon les revendications 11 à 19 pour une utilisation dans le nettoyage et la désinfection de surfaces dans un environnement domestique et/ou des zones de service alimentaire, des hôpitaux.
